# EUROPEAN PATENT APPLICATION

(11) **EP 4 599 738 A1**
(43) Date of publication of application: **13.08.2025**
(21) Application number: 24156208.1
(22) Date of filing: 07.02.2024
(51) Int. Cl.: A47G 21/02, A47J 43/28, A47G 21/10, B25B 9/02

(54) **EATING UTENSIL**

(71) Applicant: Demos Østerud, Sigurd Erik, 242 31 Hörby (SE)
(72) Inventor: Demos Østerud, Sigurd Erik, 242 31 Hörby (SE)
(74) Representative: AWA Sweden AB

(57) **Abstract**

The present disclosure relates to an eating utensil (1) comprising: a body (2) having a longitudinal axis and comprising a first member (3) and a second member (4) arranged in parallel and extending along said longitudinal axis, wherein at least the first member (3) is flexible, the first and second member (3, 4) being connected at a first point (5) and a second point (6), the first point (5) being arranged between a distal end (2A) of said body (2) and said second point (6) as seen along said longitudinal axis, wherein a respective mid portion (3A, 4A) of each member (3, 4) extends between said first point (5) and said second point (6) and forms a handle portion (7). The utensil (1) further comprises a first set of prongs (8) and a second set of prongs (9), each set of prongs being provided on a respective end portion (4B) of the second member (4), each set of prongs (8, 9) being provided at the distal end (2A) of the body (2), wherein the eating utensil (1) is configured such that compression of the handle portion (7) forces the mid portion (3A) of the first member (3) towards the mid portion (4A) of the second member (4) so as to displace the first set of prongs (8) around the first point (5) and from the second set of prongs (9).

## Description

### FIELD OF INVENTION

The present invention relates to eating utensils.

### BACKGROUND OF THE INVENTION

The choice of eating utensil is often based on the food to be eaten and how the food is served. Some of the most common set of utensils, such as a knife combined with either a fork or a spoon, although displaying great versatility, have some drawbacks. Firstly, any one utensil is not suited for picking up any type of food item. Secondly, a set of utensils require the use of both hands, which might be difficult in some situations. This could be due to disabilities, or just the fact that the other hand is occupied, e.g. by holding the plate, such as at a mingle party. There are some eating utensils that only requires one hand, such as chop sticks. However, these could be hard to manoeuvre and might require some training before comfortable and effective usage is obtained.

Hence there is a need for an improved eating utensil.

### SUMMARY OF THE INVENTION

It is thus in view of the above a need for an improved eating utensil that is adaptable in dealing with the aforementioned problems.

According to a first aspect of the present invention, an eating utensil comprising: a body having a longitudinal axis and comprising a first member and a second member arranged in parallel and extending along the longitudinal axis, wherein at least the first member is flexible, the first and second member being connected at a first point and a second point, the first point being arranged between a distal end of the body and the second point as seen along the longitudinal axis, wherein a respective mid portion of each member extending between the first point and the second point forms a handle portion; and a first set of prongs comprising at least one prong and a second set of prongs comprising at least one prong, the first set of prongs being provided on a respective end portion of the first member and the second set of prongs being provided on a respective end portion of the second member, each set of prongs being provided at the distal end of the body; wherein the eating utensil is configured such that compression of the handle portion forces the mid portion of the first member towards the mid portion of the second member so as to displace the first set of prongs around the first point and from the second set of prongs.

Since compression of the handle portion displaces at least the first set of prongs in relation to the second set of prongs, the first point act as a fulcrum facilitating pivoting motion of the first set of prongs whereby a larger distance between the first set of prongs and the second set of prongs is achieved. Thus, a larger space in which a piece of food may be received is formed. Hence, food items that are difficult to pierce or scoop can instead be gripped by release of the handle portion, making it easier to pick up such food items with a single utensil. Hereby, a multi-functional eating utensil is provided.

The second member may also be flexible. Thus, compression of the handle portion forces the respective mid portions of the first member and the second member towards each other so as to displace the first set of prongs and the second set of prongs around the first point and away from each other. Hence, the distance between the first set of prongs and the second set of prongs, created by the pivoting motion around the first point, will be further increased. Therefore larger pieces of food may be received and gripped by the eating utensil.

The eating utensil may be made of, or comprise, any suitable material, such as metal, wood or polymeric material, such as acrylic, PET, polypropylene, polycarbonate, polyethylene, polystyrene, epoxy, and/or a biodegradable polymeric material. It is to be understood that depending on choice of material or materials, any flexible member is to be dimensioned in a suitable manner.

The eating utensil may e.g. be made in an extrusion process, or by 3d-printing.

The eating utensil may have any suitable length, or material thickness. Further, the first and second members may be of equal, or different, lengths. Furthermore, the first and second members may have equal, or different, material thickness. For example, the first member may have a smaller material thickness, i.e. be thinner, than the second member, such that the first member is flexible, or at least more flexible than the second member.

The eating utensil may be configured such that the body further comprises a scoop portion extending between the first point and the prongs. Hereby, the utensil is provided with a curvature as seen transverse to the longitudinal axis of the body so as to facilitate ladling of food items.

The first point is distanced from the second point as seen along the longitudinal axis. The mid portion of each member may fully or partially extend between the first and second point. Hence, the handle portion may also fully or partially extend between the first and second point. It is to be understood that the extension of the mid portion and/or the distance between the first point and the second point is to be chosen in relation to the material and material thickness of the members such that compression of the handle portion is feasible.

The first and second member may be connected to each other in a variety of ways. For example, the first and second member may be fixedly connected at the first and/or the second point. Hereby, the structural integrity of the eating utensil is improved. The first and the second member may e.g. be integrally formed such that they are fixedly connected at the first and/or the second point. Additionally or alternatively, the first and second member may be fixedly connected to each other by, e.g., means of adhesion or a locking pin.

Alternatively, at the first point and/or the second point, the first member may comprise one of a receiving portion and an engagement portion, and the second member may comprise a complementary receiving portion or engagement portion, wherein the engagement portion is releasably connected to the receiving portion. Hereby, the first member and the second member may be selectively connected and disconnected at the first point and/or the second point. The first and second member may be fixedly connected at the first point, and releasably connected at the second point or vice versa. Alternatively, both the first point and the second point may be releasably connected, or conversely, fixedly connected. If both the first point and the second point are releasably connected, the first member and the second member may be selectively connected and disconnected. Hereby, the members may be stored and/or transported in a disconnected, more space-efficient, manner. It is also possible to replace parts when needed, instead of discarding the whole eating utensil.

The receiving portion may comprise a groove and the engagement portion may comprise a protrusion. Alternatively, the receiving portion may comprise a cut-out arranged in a side edge of the respective member, and the engagement portion may comprise a projecting portion arranged on a side edge of the respective member and extending transverse to the longitudinal axis of the body, and wherein the cut-out is provided with a pin-hole and the projecting portion is provided with a pin, the pin extending transverse to the projecting portion and being rotatably arranged in the pin-hole. Furthermore, any member may comprise a cut-out on two opposite side edges. The pin-hole may thus be a through hole, extending from the cut-out in one side edge, to the cut-out of the opposite side edge. Similarly, any member may comprise a first and a second projecting portion arranged on two opposite side edges such that the pin may extend between the projecting portions. Moreover, the pin extending between the projecting portions may be arranged in the through hole.

The projecting portion may have a semi-circular shape, wherein the cut-out has a shape complementary to the projecting portion.

The prongs of the first set and the second set of prongs may have a variety of shapes. For example, the prong in the first set of prongs, neighboring the second set of prongs, and the prong in the second set of prongs neighboring the first set of prongs may be arranged against each other, wherein the prongs are shaped so as to form a spoon-shaped structure. Alternatively the prongs may be tine-shaped. Further, the first set of prongs and the second set of prongs may comprise different numbers of prongs. Each set of prongs may, e.g., comprise at least two prongs. Alternatively, the first set of prongs may comprise at least one prong, while the second set of prongs may comprise at least two prongs and vice versa. In other words, the number of prongs in each set may vary and may differ from each other. For at least one of the first set and the second set of prongs, the prong neighboring the other set of prongs may be provided with teeth on a side surface thereof, the side surface facing the other set of prongs. In other words, in the first set of prongs, the prong being adjacent to the second set of prongs may be provided with teeth. Additionally or alternatively, the corresponding prong in the second set of prongs, being adjacent to the first set of prongs may also be provided with teeth. Thus, when the handle portion is released in order to grip a food item, the teeth will provide increased friction and a larger contact surface which improves the ability of gripping the food item.

A respective second end portion of each member may extend from the second point to a second distal end of the body, the second distal end being opposite to the distal end as seen along the longitudinal axis, the respective second end portion may form a clamping portion and wherein the eating utensil may be further configured such that compression of the handle portion forces the mid portion of the first member towards the mid portion of the second member so as to actuate the clamping portion. Actuating the clamping portion is to be understood as displacing the second end portion of the first member around the second point and from the second end portion of the second member. The clamping portion may be provided with teeth. The teeth provide increased friction and a larger contact surface which improves the ability of gripping food.

According to a second aspect of the present invention, a kit comprising a first and a second member is provided, wherein at least the first member is flexible, each member on a respective end portion thereof being provided with a respective set of prongs, each set of prongs comprising at least one prong, wherein the first and second member are releasably connectable to each other so as to form an eating utensil in accordance with the first aspect of the present invention.

At least some of the technical effect and benefits discussed in relation to the first aspect of the present invention are applicable to the second aspect of the present invention.

Hereby, the members may, in a disconnected state, be stored and/or transported in a more space-efficient manner. The ability to disconnect the members also makes it possible to replace damaged parts, instead of discarding the whole kit.

Any member may e.g. be made of, or comprise, metal, wood or polymeric material, such as biodegradable polymeric material. The first member may be made of a first material, and the second member may be made of a second material, the first material being different from the second material. That is, the first member may be made of, or comprise, wood, whereas the second member may be made of, or comprise, metal, or vice versa.

### BRIEF DESCRIPTION OF THE DRAWINGS

For exemplifying purposes, the invention will be described in closer detail in the following with reference to exemplary embodiments thereof illustrated in the attached drawings, wherein:
Fig. 1 is a perspective view of the eating utensil according to the first aspect of the present invention;
Fig. 2 is a perspective view illustrating the actuation of the eating utensil in Fig 1;
Fig. 3 is a perspective view of a variant of the eating utensil in Fig. 1;
Fig. 4 is a perspective view of a variant of the receiving portion and the engagement portion of the eating utensil in Fig. 3;
Fig. 5 is a perspective view of a variant of the eating utensil highlighting a variant of the receiving portion and the engagement portion;
Fig. 6A-6B is a top view of the eating utensil of Fig. 3 in a disassembled state, and an assembled state, respectively;
Fig 7. is a perspective view illustrating the actuation of the eating utensil in Figs. 3 and 4.

### DETAILED DESCRIPTION

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

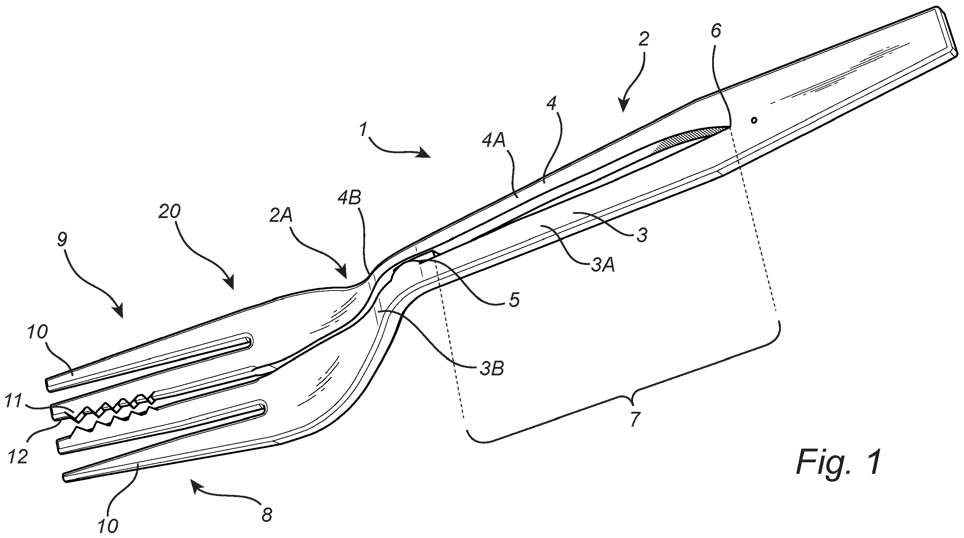

Fig. 1 is a perspective view of the eating utensil 1 according to the first aspect of the present invention. The eating utensil 1 comprises a body 2 having a longitudinal axis. The body 2 comprises a first member 3 and a second member 4. The members 3, 4 are arranged in parallel and extending along the longitudinal axis. The first member 3 and the second member 4 are connected at a first point 5 and a second point 6. The first point 5 is arranged between a distal end 2A of the body 2 and the second point 6 as seen along the longitudinal axis.

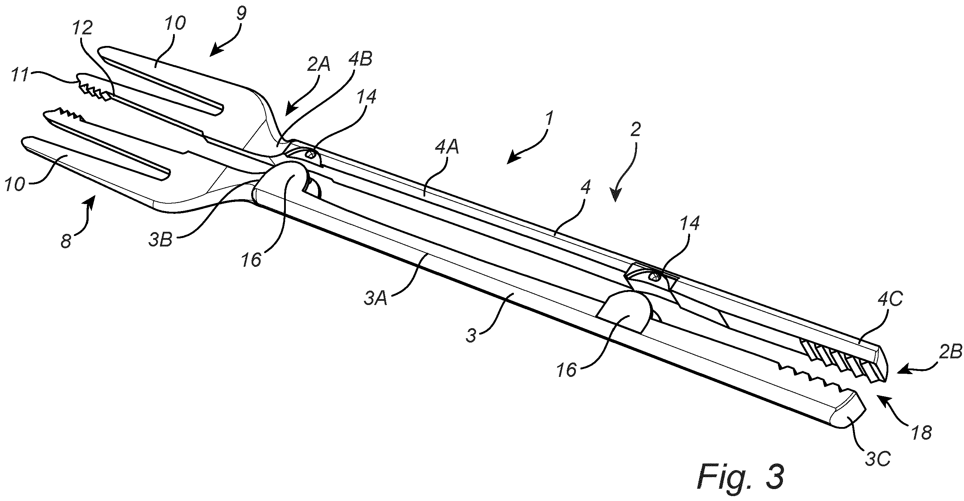

Here, the first member 3 and the second member 4 are integrally formed such that the first member 3 is fixedly connected to the second member 4 at the second point 6. However, the first member 3 is releasably connected to the second member 4 at the first point 5. Exemplary means by which the members 3, 4 are releasably connected to each other are further discussed in relation to Figs. 3 to 6.

The members 3, 4 further comprises a respective mid portion 3A, 4A, each mid portion 3A, 4A extending between the first point 5 and the second point 6 and forming a handle portion 7. The handle portion 7 is thus comprised of a portion of each member 3, 4, i.e. the respective mid portions 3A, 4A, which are separated from, or otherwise related to, each other such that they may be pressed towards each other. In Fig. 1, the mid portions 3A, 4A are separated by a gap. However, the mid portions 3A, 4A may be separated by, e.g., compressible material, such as thin, foldable material or dampening material. Here, the two members 3, 4 are separated along a majority of the longitudinal extension of the eating utensil 1, and are only connected at the first point 5 and the second point 6. That is, the members 3, 4 are separated from each other between the first point 5 and the distal end 2A.

The eating utensil 1 further comprises a first set of prongs 8 and a second set of prongs 9. The first set of prongs 8 is provided at an end portion 3B of the first flexible member 3, wherein the second set of prongs 9 is provided at an end portion 4B of the second flexible member 4. Here, both the first set of prongs 8 and the second set of prongs 9 each comprises two prongs 10. However, the two sets of prongs 8, 9 may comprise different numbers of prongs 10. For example, the first set of prongs 8 may comprise one prong 10, while the second set of prongs 9 comprises two prongs 10, or vice versa. In other words, the number of prongs in each set may vary and may differ from each other. Here, the prongs 10 are arranged next to each other and substantially in a plane. Here, for each set of prongs 8, 9, the prong 10 neighboring the other set of prongs are provided with teeth 11 on a side surface 12 thereof. Each side surface 12 is thereby facing the other set of prongs 8, 9.

Here, the eating utensil 1 further comprises a scoop portion 20 extending between the first point 5 and the set of prongs 8, 9. Hereby, the utensil 1 is provided with a curvature as seen transverse to the longitudinal axis of the body 2 so as to facilitate ladling of food items.

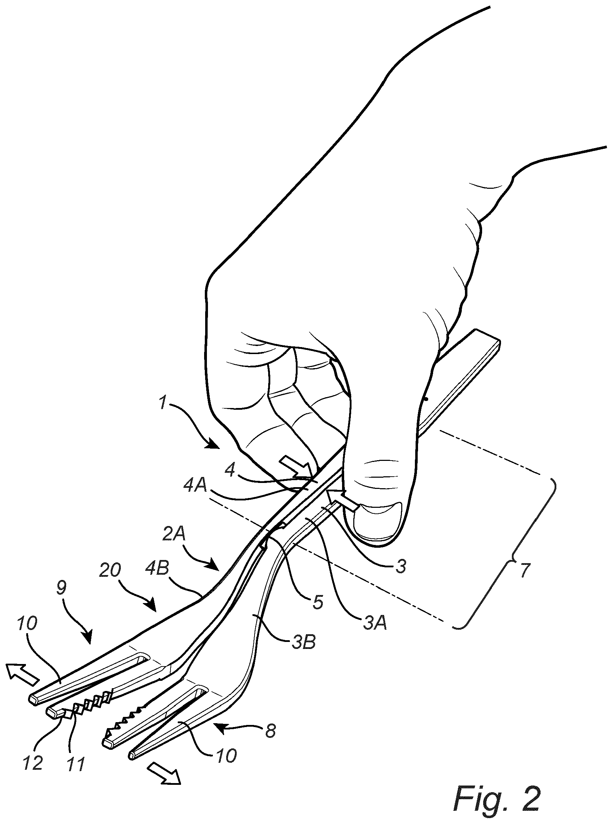

Actuation of the eating utensil 1 is shown in Fig. 2. The eating utensil 1 is configured such that compression of the handle portion 7 forces the mid portion 3A of the first member 3 towards the mid portion 4A of the second member 4. Hereby, the first set of prongs 8 is displaced around the first point 5 and from the second set of prongs 9. This is due to at least the first member 3 being flexible. However, as is seen in Fig. 2, the second member 4 may also be flexible. Thus, compression of the handle portion 7 forces the respective mid portions 3A, 4A of the first member 3 and the second member 4 towards each other so as to displace the first set of prongs 8 and the second set of prongs 9 around the first point 5 and away from each other. The eating utensil 1 may be made of, or comprise, any suitable material, such as metal, wood or polymeric material, such as biodegradable polymeric material. It is to be understood that depending on choice of material or materials, any flexible member 3, 4 is to be dimensioned in a suitable manner. The eating utensil may have any suitable length, or material thickness. Further, the first and second members 3, 4 may be of equal, or different, lengths. Furthermore, the first and second members 3, 4 may have equal, or different, material thickness. For example, the first member 3 may have a smaller material thickness, i.e. be thinner, than the second member 4, such that the first member 3 is flexible, or at least more flexible than the second member 4. It is to be understood that the eating utensil 1 may comprise further members, such as a third member. A third member may e.g. be arranged between the first member 3 and the second member 4.

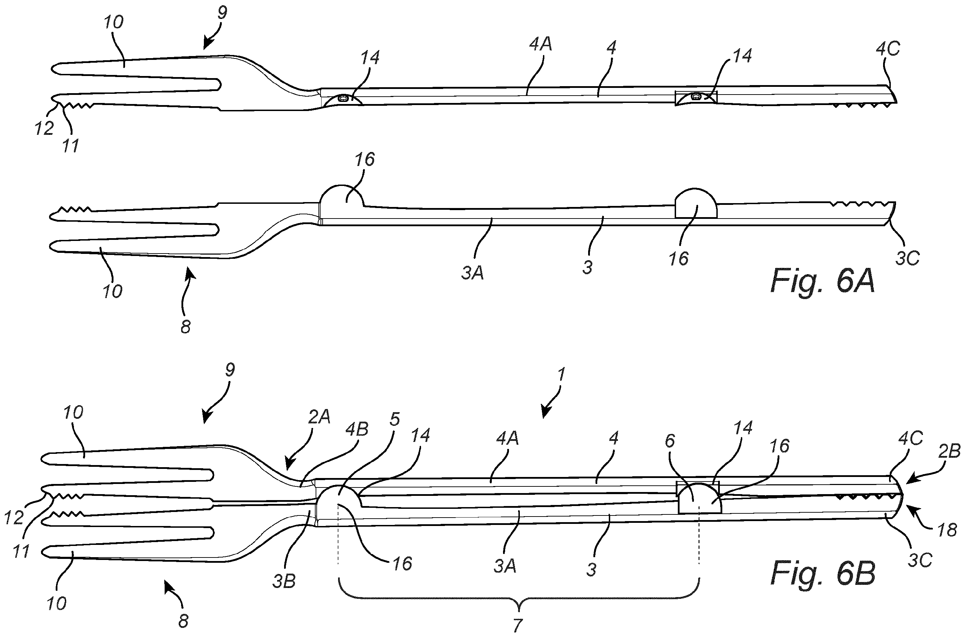

In Fig. 3, a variant of the eating utensil 1 is shown. Here, the utensil 1 is provided as a kit comprising a first and a second member 3, 4. Here, the first and second member 3, 4 are releasably connectable to each other so as to form the eating utensil 1 (shown in Figs 6A, 6B). Here, the second member 4 comprises a first and a second receiving portion 14. The first member 3 comprises a complementary first and a second engagement portion 16. That is, each receiving portion 14 of the second member 4 is to be releasably connected with a complementary respective engagement portion 16 of the first member 3. Hereby, the first member 3 and the second member 4 may be selectively connected and disconnected.

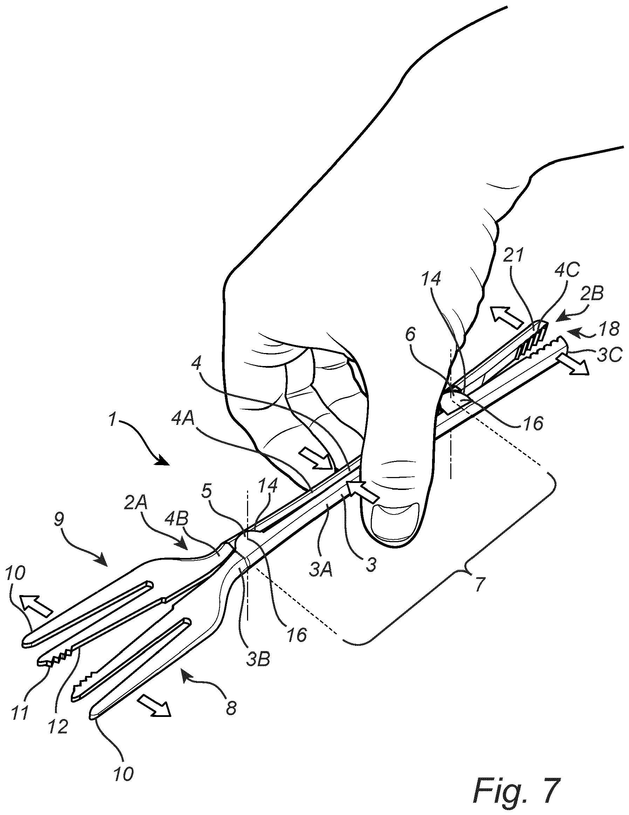

Here, a respective second end portion 3C, 4C of each member 3, 4 extends to a second distal end 2B of the body 2, the second distal end 2B being opposite to the distal end 2A as seen along the longitudinal axis. The respective second end portions 3C, 4C form a clamping portion 18. Hereby, the eating utensil 1 is further configured such that compression of the handle portion 7 (shown in Fig. 6B) forces the mid portion 3A of the first member 3 towards the mid portion 4A of the second member 4 so as to actuate the clamping portion 18, which is shown in more detail in Fig. 7.

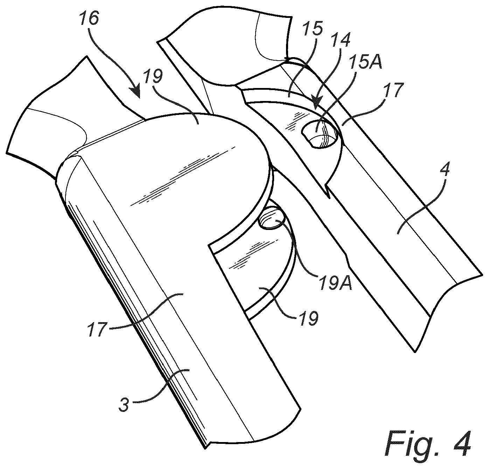

The receiving portion 14 and the engagement portion 16 are shown in more detail in Fig. 4. Here, the receiving portion 14 comprises a cut-out 15 arranged in a side edge 17 of the second member 4. The cut-out 15 is provided with a pin-hole 15A. The engagement portion 16 comprises a first and a second projecting portion 19 arranged on a respective side edge 17 of the first member 3 and extending transverse to the longitudinal axis of the body 2. The projecting portions 19 have a semi-circular shape, whereas the cut-out 15 has a shape complementary to the projecting portions 19. Each projecting portion 19 is provided with a pin 19A, the pin 19A extending transverse to the projecting portion 19 and being rotatably arrangeable in a pin-hole 15A of a receiving portion 14. Alternatively to what is shown in Fig. 4, the pin-hole 15A may be a through hole, extending from a cut-out 15 in one side edge 17 of the second member 4 to a cut-out 15 in the opposite side edge 17 of the second member 4. Similarly, the first member 3 may comprise a first and a second projecting portion 16 arranged on two opposite side edges 17 and wherein the pin 19A extends between the two projecting portions 16.

It is to be understood that the first member 3 may be provided with at least one receiving portion 14, whereas the second member 4 may be provided with at least one engagement portion 16. The first member 3 and the second member 4 may, e.g., be provided with one respective receiving portion 14 and one respective engagement portion 16, each.

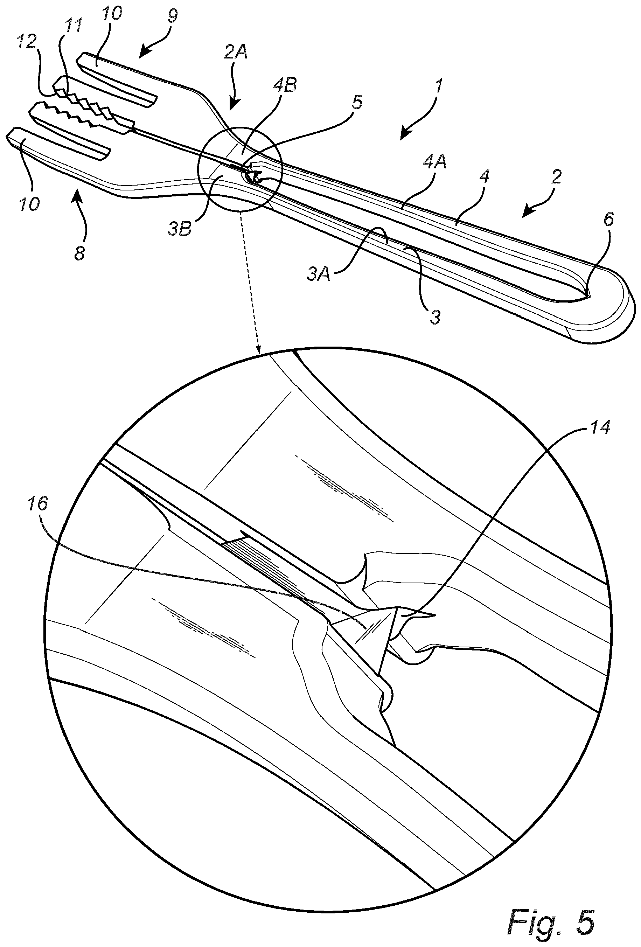

In Fig. 5, a variant of the eating utensil 1 is shown. In particular, the first member 3 and the second member 4, which are releasably connected at the first point 5, comprise a respective variant of the receiving portion 14 and the engagement portion 16. Here, the first member 3 comprises an engagement portion 16 in the form of a protrusion, whereas the second member 4 comprises a receiving portion 14 in the form of a groove. The protrusion is arranged in the groove.

In Fig. 6A, a top view of the kit in a disassembled state is shown, whereas in Fig. 6B, a top view of the kit in an assembled state, so as to form an eating utensil 1, is shown.

In Fig. 7, actuation of the eating utensil 1 as shown in Figs. 3-4 and 6A and 6B is shown. As can be seen, apart from the displacement of the first set of prongs 8 and the second set of prongs 9 around the first point 5 and away from each other, as has been described in relation to Figs. 1 and 2, the clamping portion 18 is also actuated. Actuation of the clamping portion 18, as shown, entails that the second end portion 3C of the first member 3 is discplaced around the second point 6 and from the second end portion 4C of the second member 4. Simultaneously, since both members 3, 4 here are flexible, the second end portion 4C of the second member 4 is displaced around the second point 6 and from the second end portion 3C of the first member 3. Furthermore, the clamping portion 18 is here provided with teeth 21. The teeth 21 provide increased friction and a larger contact surface which improves the ability of gripping food.

## Claims

1. An eating utensil (1) comprising:
a body (2) having a longitudinal axis and comprising a first member (3) and a second member (4) arranged in parallel and extending along said longitudinal axis, wherein at least the first member (3) is flexible, the first and second member (3, 4) being connected at a first point (5) and a second point (6), the first point (5) being arranged between a distal end (2A) of said body (2) and said second point (6) as seen along said longitudinal axis, wherein a respective mid portion (3A, 4A) of each member (3, 4) extending between said first point (5) and said second point (6) forms a handle portion (7); and
a first set of prongs (8) comprising at least one prong (10) and a second set of prongs (9) comprising at least one prong (10), the first set of prongs (8) being provided on a respective end portion (3B) of the first member (3) and the second set of prongs (9) being provided on a respective end portion (4B) of the second member (4), each set of prongs (8, 9) being provided at the distal end (2A) of the body (2); wherein
the eating utensil (1) is configured such that compression of the handle portion (7) forces the mid portion (3A) of the first member (3) towards the mid portion (4A) of the second member (4) so as to displace the first set of prongs (8) around the first point (5) and from the second set of prongs (9).

2. The eating utensil (1) according to claim 1, wherein at the first point (5) and/or the second point (6), the first member (3) comprises one of a receiving portion (14) and an engagement portion (16), and the second member (4) comprises a complementary receiving portion (14) or engagement portion (16), wherein the engagement portion (16) is releasably connected to the receiving portion (14).

3. The eating utensil (1) according to claim 2 wherein the receiving portion (14) comprises a groove and the engagement portion (16) comprises a protrusion.

4. The eating utensil (1) according to claim 2 wherein the receiving portion (14) comprises a cut-out (15) arranged in a side edge (17) of the respective member (3, 4), and the engagement portion (16) comprises a projecting portion (19) arranged on a side edge (17) of the respective member (3, 4) and extending transverse to the longitudinal axis of the body (2), and wherein the cut-out (15) is provided with a pin-hole (15A) and the projecting portion (19) is provided with a pin (19A), the pin (19A) extending transverse to said projecting portion (19) and being rotatably arranged in said pin-hole (15A).

5. The eating utensil (1) according to claim 4 wherein the projecting portion (19) has a semi-circular shape and wherein said cut-out (15) has a shape complementary to said projecting portion (19).

6. The eating utensil (1) according to claim 1, wherein the first and second member (3, 4) are fixedly connected at the first and/or the second point (5, 6).

7. The eating utensil (1) according to any preceding claim wherein a respective second end portion (3C, 4C) of each member (3, 4) extends from said second point (6) to a second distal end of the body (2B), said second distal end (2B) being opposite to the distal end (2A) as seen along the longitudinal axis, the respective second end portions (3C, 4C) forming a clamping portion (18) and wherein the eating utensil (1) is further configured such that compression of the handle portion (7) forces the mid portion (3A) of the first member (3) towards the mid portion (4A) of the second member (4) so as to actuate the clamping portion (18).

8. The eating utensil (1) according to claim 6 wherein the clamping portion (18) is provided with teeth (21).

9. The eating utensil (1) according to any preceding claim, wherein the body (2) further comprises a scoop portion (20) extending between the first point (5) and the first and second set of prongs (8, 9).

10. The eating utensil (1) according to any preceding claim wherein for at least one of the first set and the second set of prongs (8, 9), the prong (10) neighboring the other set of prongs (8, 9) is provided with teeth (11) on a side surface (12) thereof, said side surface (12) facing said other set of prongs (8, 9).

11. The eating utensil (1) according to any preceding claim wherein the utensil (1) is made of metal.

12. The eating utensil (1) according to any preceding claim wherein the utensil (1) is made of wood.

13. The eating utensil (1) according to any preceding claim wherein each set of prongs (8, 9) comprises at least two prongs (10).

14. A kit comprising a first and a second member (3, 4), wherein at least the first member (3) is flexible, each member (3, 4) on a respective end portion (3B, 4B) thereof being provided with a respective set of prongs (8, 9), each set of prongs (8, 9) comprising at least one prong (10), wherein the first and second member (3, 4) are releasably connectable to each other so as to form an eating utensil (1) in accordance to claim 1.

15. A kit according to claim 14 wherein the first member (3) is made of a first material, and the second member (4) is made of a second material, the first material being different from the second material.
